# FASCICULE DE BREVET EUROPEEN

(11) **EP 2 836 243 B1**
(45) Date de publication et mention de la délivrance du brevet: **17.02.2016**
(21) Numéro de dépôt: 13718533.6
(22) Date de dépôt: 15.04.2013
(51) Int. Cl.: A61L 26/00

(54) **POUDRE FORMANT PANSEMENT ET MATERIAU ABSORBANT CORRESPONDANT**
PULVER ZUR HERSTELLUNG EINES WUNDVERBANDES UND ENTSPRECHENDES ABSORBIERENDES MATERIAL
POWDER FORMING A DRESSING AND CORRESPONDING ABSORBING MATERIAL

(30) Priorité: 13.04.2012 FR 1253452
(43) Date de publication de la demande: 18.02.2015
(73) Titulaire: Les Laboratoires Brothier, 92000 Nanterre (FR)
(72) Inventeur: LACK, Stéphane, F-49070 Beaucouze (FR); BARIKOSKY, Michel, F-75007 Paris (FR); GIRARDIERE, Christian, F-75116 Paris (FR)
(74) Mandataire: Gevers & Orès
(86) Numéro de dépôt international: PCT/EP2013/057771
(87) Numéro de publication internationale: WO 2013/153225

(56) Documents cités:
- EP-A1- 1 252 901
- WO-A2-98/28013
- FR-A1- 2 671 486

## Description

Le domaine de l'invention est celui des pansements.

L'invention s'intéresse plus particulièrement aux poudres utilisées pour l'hémostase et la réparation tissulaire, par exemple pour panser les plaies.

Une plaie provoque une perte de substance ou de liquide biologique (sang ou exsudat). Appliquée sur la plaie, une poudre hémostatique, cicatrisante, absorbe le liquide biologique qui suinte, ou qui exsude, et permet de stopper le saignement par action mécanique et/ou physiologique. L'état divisé de la poudre permet un contact optimal avec le tissu de la plaie, notamment dans le cas des plaies anfractueuses ou cruentées. Par la suite, une bonne régénération tissulaire est obtenue, entre autres, par la séquestration de l'exsudat et des toxines, un maintien de l'humidité, un échange gazeux optimal et une protection contre la surinfection.

Le document EP1252901 A divulgue un pansement d'alginate de calcium qui se présente sous une forme pulvérulente.

Les poudres de ce type sont ainsi destinées à être appliquées sur des plaies humides, qui exsudent, des escarres, des ulcères variqueux, des plaies postopératoires, par exemple.

Malheureusement, les poudres hémostatiques existantes ont parfois un pouvoir d'absorption et de rétention du liquide biologique insuffisant. En effet, suite à leur application sur une plaie, ces poudres, constituées généralement de billes sphériques d'un matériau hémostatique, forment une pâte qui s'effondre rapidement, limitant ainsi leur pouvoir d'absorption et de rétention. De plus cette pâte peut limiter les échanges gazeux au niveau de la plaie.

La Demanderesse a donc cherché à proposer une poudre ayant un fort pouvoir d'absorption et de rétention.

Ainsi, l'invention de la présente demande concerne tout d'abord une poudre formant pansement, caractérisée par le fait qu'elle est formée d'une pluralité de particules de forme cylindrique obtenues à partir de fibres.

La poudre de l'invention est avantageusement une poudre hémostatique obtenue à partir de fibres hémostatiques.

De manière remarquable, suite à son application sur une plaie, les particules de la poudre de l'invention forment une structure tridimensionnelle les répartissant géométriquement de manière aérée sur la surface de la plaie. Ainsi, grâce à la forme cylindrique particulière des particules de la poudre, son pouvoir d'absorption et de rétention est notablement accru tout en assurant l'échange gazeux avec la plaie.

Maintenant, si on considère que les fibres courtes, ou flocs, qu'on dépose dans la trémie du système décrit dans FR 2671486 forment une poudre au sens de la présente invention, alors la demanderesse entend revendiquer l'usage de cette poudre comme moyen pour panser directement les plaies.

Dans ce cas, l'activité inventive serait tout aussi manifeste. En effet, à l'époque du dépôt du document ci-dessus, la demanderesse avait finalement la poudre de l'invention toute prête à l'usage et ne l'a ni vu ni eu l'audace de la tester en tant que telle. Ce ne serait que vingt ans après qu'elle s'en serait aperçue. Ainsi, l'invention concerne également l'utilisation de la poudre de l'invention, telle quelle, en tant que pansement destiné à l'hémostase ou à la réparation de tissus biologiques lésés.

Avantageusement, les particules ont une longueur comprise entre 20 µm et 2 mm et un diamètre compris entre 5 µm et 50 µm. De préférence, ce diamètre est compris entre 10 µm et 30 µm.

En effet, les particules dont le diamètre est inférieur à 5 µm forment des aérosols. Leur diffusion broncho-pulmonaire présente une toxicité respiratoire induite de type emphysème. Par ailleurs, au-delà de 50 µm, on constate une limitation de la capacité d'écoulement et d'adhérence aux tissus.

De préférence, les fibres sont en alginate de calcium.

En effet, par la gélification de sa structure au contact du sang ou des exsudats d'une plaie suintante, l'alginate de calcium crée une sorte de peau artificielle qui protège la plaie des chocs extérieurs et des variations thermiques.

L'invention concerne également un procédé de fabrication d'une poudre hémostatique comprenant les étapes de :
- préparation d'une solution d'alginate de sodium ;
- immersion de la solution dans un bain de chlorure de calcium pour former une fibre d'alginate de calcium ;
- lavage et séchage de la fibre ; et
- hachage de la fibre pour obtenir des particules de forme cylindrique.

Avantageusement, le procédé comprend une étape de bobinage de la fibre avant son hachage.

L'invention concerne aussi une éponge comprenant une poudre selon l'invention.

L'utilisation de l'éponge de l'invention facilite le dépôt de la poudre sur une plaie chirurgicale interne sans utiliser d'ancillaire spécifique. Lorsqu'elle comprend une poudre hémostatique, cette éponge permet une absorption immédiate des exsudats de la plaie.

Avantageusement, l'éponge est biorésorbable et biocompatible.

De par sa nature biorésorbable et biocompatible, l'éponge peut rester in-situ après une intervention chirurgicale.

De préférence, l'éponge comprend au moins un biopolymère.

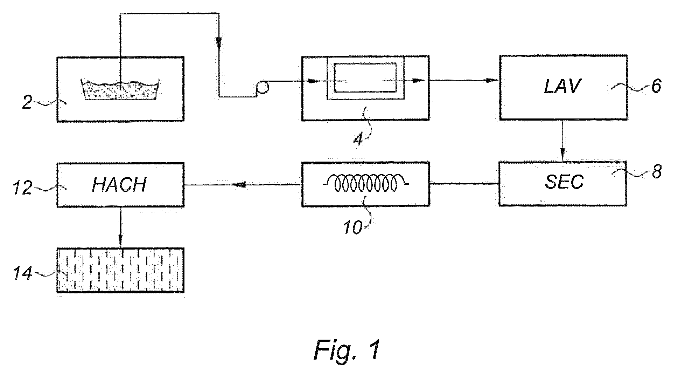

Un mode de réalisation de l'invention va maintenant être décrit de façon plus précise mais non limitative en regard du dessin annexé comprenant une figure unique 1 illustrant les étapes successives du procédé de fabrication d'une poudre hémostatique à base de fibres d'alginate de calcium.

Lors d'une étape 2, une solution d'alginate de sodium est préparée par immersion d'une poudre d'alginate de sodium dans une cuve ou un réacteur contenant de l'eau pendant une heure, par exemple.

A l'étape 4, la solution d'alginate de sodium traverse une filière dans un bain de chlorure de calcium pour former une fibre d'alginate de calcium.

A l'étape 6, la fibre d'alginate de calcium est lavée dans de l'eau purifiée afin d'éliminer les ions calcium et chlorure en excès. Selon l'analyse des rejets, plusieurs lavages successifs peuvent être nécessaires.

A l'étape 8, la fibre d'alginate de calcium est séchée par évaporation de l'eau. Ce séchage est réalisé ici en chauffant la fibre modérément à une température comprise entre 40°C et 45°C pour ne pas la dégrader.

A l'étape 10, la fibre est enroulée en bobine.

A l'étape 12, la fibre est hachée, par exemple par broyage et tamisage, pour obtenir une poudre 14 formée de particules de forme cylindrique ayant une longueur comprise entre 20 µm et 2 mm et un diamètre compris entre 10 µm et 30 µm.

Cette poudre 14 peut être utilisée telle quelle en tant que pansement destiné à l'hémostase de tissus biologiques lésés ou à la réparation tissulaire ou osseuse ou de cartilages, l'alginate de calcium ayant des propriétés hémostatiques et de réparation.

De manière avantageuse, la poudre 14 peut également être utilisée pour fabriquer une éponge médicale biorésorbable et biocompatible. Pour cela, la poudre 14 est incorporée dans une mousse formée d'au moins un biopolymère, qui peut être soit un dérivé cellulosique (comme la CarboxyMethylCellulose (CMC) à titre d'exemple) soit un ester d'alginate (comme le Propylène Glycol Alginate (PGA), à titre d'exemple), et d'un mélange d'agents moussants, stabilisants et absorbants. Cette mousse est ensuite lyophilisée pour former l'éponge et servir de matériau absorbant porteur de la poudre hémostatique.

Les agents moussants utilisés pour l'invention sont essentiellement des tensio-actifs tels que le poloxamère, le chlorure de benzalkonium ou l'ammonium lauryl sulfate. Les stabilisants sont choisis parmi le carbopol ou des sels de métaux divalents et l'absorbant parmi l'alginate de sodium ou le pullulane. Par ailleurs l'agent moussant peut aussi être stabilisant. Bien entendu, d'autres modes de réalisation sont envisageables.

Cette poudre hémostatique en alginate de calcium, ou une variante, peut être adaptée à la réparation tissulaire ou osseuse ou à la réparation de cartilages.

## Revendications

1. Poudre hémostatique (14) formant un pansement, **caractérisée par le fait qu'**elle est formée d'une pluralité de particules de forme cylindrique obtenues à partir de fibres.

2. Poudre selon la revendication 1, dans laquelle les particules ont une longueur comprise entre 20 µm et 2 mm et un diamètre compris entre 5 µm et 50 µm.

3. Poudre selon la revendication 1 ou 2, dans laquelle les fibres sont des fibres hémostatiques.

4. Poudre selon la revendication 3, dans laquelle les fibres sont en alginate de calcium.

5. Procédé de fabrication d'une poudre hémostatique (14) comprenant les étapes de :
- préparation (2) d'une solution d'alginate de sodium ;
- immersion (4) de la solution dans un bain de chlorure de calcium pour former une fibre d'alginate de calcium ;
- lavage (6) et séchage (8) de la fibre ; et
- hachage (12) de la fibre pour obtenir des particules de forme cylindrique.

6. Procédé de fabrication selon la revendication 4, comprenant une étape de bobinage (10) de la fibre avant son hachage (12).

7. Poudre selon l'une quelconque des revendications 1 à 4 formant un pansement pour l'utilisation dans l'hémostase ou la réparation de tissus biologiques lésés.

8. Matériau absorbant comprenant une poudre hémostatique selon l'une quelconque des revendications 1 à 4.

9. Matériau absorbant selon la revendication 8, **caractérisée par le fait qu'**il est biorésorbable et biocompatible.

10. Matériau absorbant selon la revendication 9, comprenant au moins un biopolymère.

11. Matériau absorbant selon l'une des revendications 8 à 10 en tan que pansement pour l'utilisation dans l'hémostase ou la réparation de tissus biologiques lésés.

12. Procédé de réalisation d'un matériau absorbant comprenant une poudre hémostatique selon l'une des revendications 1 à 4 comportant les étapes suivantes :
- incorporation de la poudre dans une mousse formée d'au moins un biopolymère et d'au moins un agent moussant, stabilisant et/ou absorbant,
- lyophilisation de la mousse pour former ledit matériau absorbant sous une forme d'éponge.

13. Procédé selon la revendication 12 dans lequel le biopolymère est un dérivé cellulosique ou un ester d'alginate.

## Patentansprüche

1. Hämostatisches Pulver (14) zur Herstellung eines Wundverbandes, **dadurch gekennzeichnet, dass** es aus einer Vielzahl von Partikeln mit zylindrischer Form, die aus Fasern erhalten werden, gebildet ist.

2. Pulver nach Anspruch 1, wobei die Partikel eine Länge im Bereich zwischen 20 µm und 2 mm und einen Durchmesser im Bereich zwischen 5 µm und 50 µm aufweisen.

3. Pulver nach Anspruch 1 oder 2, wobei es sich bei den Fasern um hämostatische Fasern handelt.

4. Pulver nach Anspruch 3, wobei die Fasern aus Calciumalginat sind.

5. Verfahren zur Fertigung eines hämostatischen Pulvers (14), das die folgenden Schritte umfasst:
- Herstellen (2) einer Natriumalginatlösung;
- Eintauchen (4) der Lösung in ein Calciumchloridbad, um eine Calciumalginatfaser zu bilden;
- Waschen (6) und Trocknen (8) der Faser; und
- Zerkleinern (12) der Faser, um Partikel mit zylindrischer Form zu erhalten.

6. Herstellungsverfahren nach Anspruch 4, das einen Schritt zum Aufwickeln (10) der Faser vor deren Zerkleinerung (12) umfasst.

7. Pulver nach einem der Ansprüche 1 bis 4 zur Herstellung eines Wundverbands zur Verwendung bei der Hämostase oder der Reparatur von verletzten biologischen Geweben.

8. Absorbierendes Material, umfassend ein hämostatisches Pulver nach einem der Ansprüche 1 bis 4.

9. Absorbierendes Material nach Anspruch 8, **dadurch gekennzeichnet, dass** es bioresorbierbar und biokompatibel ist.

10. Absorbierendes Material nach Anspruch 9, umfassend mindestens ein Biopolymer.

11. Absorbierendes Material nach einem der Ansprüche 8 bis 10 als Wundverband zur Verwendung bei der Hämostase oder der Reparatur von verletzten biologischen Geweben.

12. Verfahren zur Ausführung eines absorbierenden Materials, umfassend ein hämostatisches Pulver nach einem der Ansprüche 1 bis 4, das die folgenden Schritte umfasst:
- Vermengen des Pulvers mit einem Schaum, der aus mindestens einem Biopolymer und mindestens einem Schaumbildner, Stabilisator und/oder Absorptionsmittel gebildet ist,
- Lyophilisieren des Schaums, um das absorbierende Material in Form eines Schwamms zu bilden.

13. Verfahren nach Anspruch 12, wobei das Biopolymer ein Cellulosederivat oder ein Alginatester ist.

## Claims

1. A hemostatic powder (14) forming a bandage, **characterized by** the fact that it is formed with a plurality of particles of cylindrical shape obtained from fibers.

2. The powder according to claim 1, wherein the particles have a length comprised between 20 µm and 2 mm and a diameter comprised between 5 µm and 50 µm.

3. The powder according to claim 1 or 2, wherein the fibers are hemostatic fibers.

4. The powder according to claim 3, wherein the fibers are in calcium alginate.

5. A method for manufacturing a hemostatic powder (14) comprising the steps:
- preparing (2) a sodium alginate solution;
- immersing (4) the solution in a calcium chloride bath in order to form a calcium alginate fiber;
- washing (6) and drying (8) the fiber; and
- chopping (12) the fiber for obtaining particles with a cylindrical shape.

6. The manufacturing method according to claim 4, comprising a step for winding up (10) the fiber before its chopping (12).

7. The powder according to any of claims 1 to 4 forming a bandage for use in hemostasis or repair of lesioned biological tissues.

8. An absorbent material comprising a hemostatic powder according to any of claims 1 to 4.

9. The absorbent material according to claim 8, **characterized by** the fact that it is bioresorbable and biocompatible.

10. The absorbent material according to claim 9, comprising at least one biopolymer.

11. The absorbent material according to one of claims 8 to 10 as a bandage for use in in hemostasis or repair of lesioned biological tissues.

12. A method for making an absorbent material comprising a hemostatic powder according to one of claims 1 to 4, including the following steps:
- incorporating the powder in a foam formed with at least one biopolymer and at least one foaming, stabilizing and/or absorbent agent,
- freeze-drying the foam in order to form said absorbent material in a sponge-like form.

13. The method according to claim 12, wherein the biopolymer is a cellulose derivative or an alginate ester.
